# EUROPEAN PATENT SPECIFICATION

(11) **EP 4 126 099 B1**
(45) Date of publication and mention of the grant of the patent: **15.01.2025**
(21) Application number: 20717948.2
(22) Date of filing: 26.03.2020
(51) Int. Cl.: A61M 1/16, A61M 1/36

(54) **EXTRACORPOREAL BLOOD CONDITIONING DEVICES AND METHODS**
EXTRAKORPORALE BLUTKONDITIONIERUNGSVORRICHTUNGEN UND -VERFAHREN
PROCÉDÉS ET DISPOSITIFS DE CONDITIONNEMENT EXTRACORPOREL DE SANG

(43) Date of publication of application: 08.02.2023
(60) Divisional application: 24217745.9
(73) Proprietor: Sorin Group Italia S.r.l., 41037 Mirandola (MO) (IT)
(72) Inventor: ZANIBONI, Andrea, 41030 San Martino Spino (MO) (IT)
(74) Representative: Buzzi, Notaro & Antonielli d'Oulx S.p.A.
(86) International application number: PCT/IB2020/052855
(87) International publication number: WO 2021/191661

(56) References cited:
- EP-A1- 3 574 939
- EP-A2- 1 810 704
- WO-A1-2019/166823
- US-A1- 2009 180 924
- US-A1- 2012 277 653
- US-A1- 2016 095 969
- US-A1- 2018 078 695

## Description

### TECHNICAL FIELD

The present disclosure relates to extracorporeal circulation. More specifically, the present disclosure relates to extracorporeal blood conditioning devices and methods for conditioning blood in extracorporeal circulation.

### BACKGROUND

Generally, hollow fiber blood oxygenators that are used to exchange oxygen (O2) and carbon dioxide (CO2) in extracorporeal circulation during cardiac surgery have one of two forms. Either the oxygenator has a cylindrical housing with a crisscross hollow fiber mat spirally wound around a cylindrical central core, or the oxygenator has a polygonal housing with stacked (piled) hollow fiber mat layers. Blood flows outside the hollow fiber lumens and oxygenating gases flow inside the hollow fiber lumens.

In cylindrically wound oxygenators, blood flow across the hollow fibers may be radial, longitudinal, circumferential, or a combination of two or more of these. Often, this leads to a blood flow path that is relatively long and non-laminar to guarantee high gas exchange efficiency with reduced surface area and limited device priming volume, which are important parameters for optimal patient perfusion. If the blood path is too long, the resulting pre-oxygenator to post-oxygenator pressure gradient may be elevated, causing excessive mechanical stress and possible damage to blood cells. Also, at times, the pressure gradient may become so high that it is not possible to reach a desired extracorporeal blood flow rate, especially if the heart lung machine (HLM) pump, used to force blood through the extracorporeal circuit, is of the centrifugal (nonocclusive) type. Such situations may lead the perfusionist, i.e., the HLM specialist, to change out the oxygenator during operation, which is a traumatic event putting the patient at risk, as circulation needs to be momentarily interrupted. Moreover, from a manufacturing point of view, a wound oxygenator is potted with potting material (resin) at both extremities to hermetically seal and separate the blood from the gases. This potting is often done in two or more steps, which adds complexity and cost to manufacturing the oxygenator.

In stacked hollow fiber mat layer oxygenators, blood flows outside the hollow fibers in a direction that is transverse to the stacked hollow fiber mat layers. Typically, this leads to shorter blood flow paths than in the cylindrically wound oxygenator and the resulting pre-oxygenator to post-oxygenator blood pressure gradient is lower such that mechanical damage to blood cells is reduced. However, an inconvenience derives from the housing shape which is generally polygonal, requiring the potting to be polygonal and characterized by dead angles where blood can stagnate and start to clot. This may cause progressive loss of gas exchange efficiency and, sometimes, force the perfusionist to change out the oxygenator during operation, which is, as already explained, a traumatic event as circulation needs to be momentarily interrupted. Moreover, polygonal fiber potting is often performed in more than two steps, which adds complexity and cost to manufacturing the oxygenator.

For the above reasons, manufacturers continue to improve the oxygenator devices.

Relevant prior art is for instance disclosed in documents US2009/180924 A1, WO2019/166823 A1, US2012/277653 A1, EP3574939 A1 and EP1810704 A2.

### SUMMARY

A device for conditioning blood according to the present invention comprises the technical features of independent claim 1. Preferred embodiments thereof are as defined in dependent claims 2-8. A method of manufacturing a device for conditioning blood according to the present invention comprises the technical features of independent claim 9. Preferred embodiments thereof are as defined in dependent claims 10-15.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

Fig. 1 is a diagram illustrating a system configured to condition blood during extracorporeal circulation, in accordance with embodiments of the subject matter of the disclosure.
Fig. 2 is a diagram illustrating a top view of a modular blood conditioning device, in accordance with embodiments of the subject matter of the disclosure.
Fig. 3 is a diagram illustrating a longitudinal section view of the modular blood conditioning device, in accordance with embodiments of the subject matter of the disclosure.
Fig. 4 is a diagram illustrating the construction of a hollow fiber mat layer used in the blood conditioning device, in accordance with embodiments of the subject matter of the disclosure.
Fig. 5 is a top view diagram illustrating the components of the device piled into a potting mold and ready for potting, in accordance with embodiments of the subject matter of the disclosure.
Fig. 6 is a diagram illustrating a cross section view of the potted body of the device, in accordance with embodiments of the subject matter of the disclosure.
Fig. 7 is a diagram illustrating a cross section view of the device along the line A-A in Fig. 3, corresponding to a middle height line of the gas exchanger module, in accordance with embodiments of the subject matter of the disclosure.
Fig. 8 is a diagram illustrating an exploded view of a single module belonging to the device, in a standalone configuration, in accordance with embodiments of the subject matter of the disclosure.
Fig. 9 is a diagram illustrating an assembled view of the module of Fig. 8, in accordance with embodiments of the subject matter of the disclosure.
Fig. 10 is a diagram illustrating an assembled view of another version of the module of Fig. 9, in accordance with embodiments of the subject matter of the disclosure.
Fig. 11 is a flow chart diagram illustrating a method of manufacturing a device for conditioning blood, in accordance with embodiments of the subject matter of the disclosure.

While the disclosure is amenable to various modifications and alternative forms, specific embodiments have been shown by way of example in the drawings and are described in detail below. The intention, however, is not to limit the disclosure to the embodiments described. On the contrary, the invention is defined by the appended claims.

### DETAILED DESCRIPTION

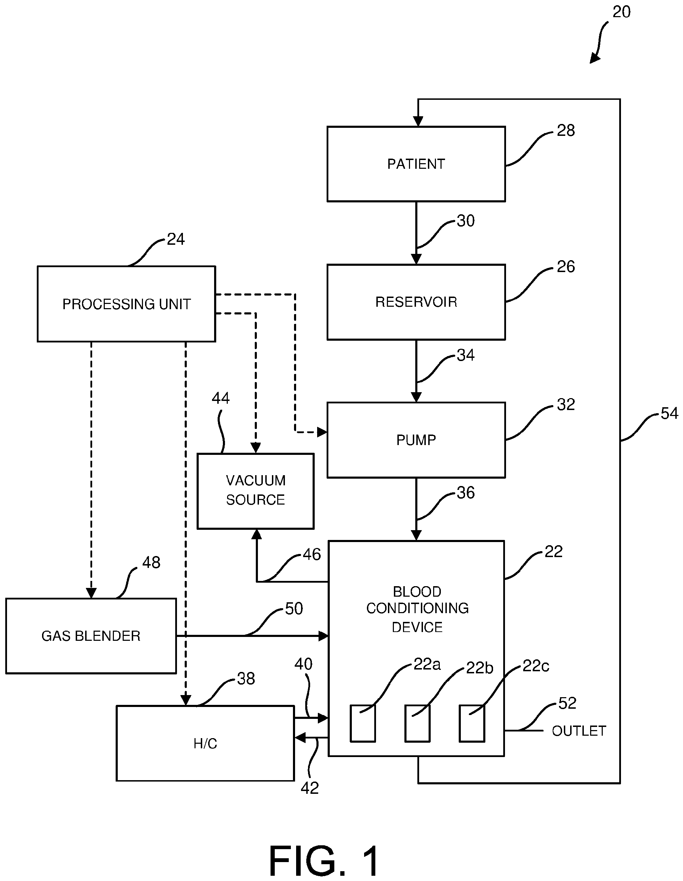

Fig. 1 is a diagram illustrating a system 20 configured to condition blood during extracorporeal circulation, in accordance with embodiments of the subject matter of the disclosure. The system 20 includes a blood conditioning device 22, also referred to as a modular blood conditioning device 22. In embodiments illustrated in Fig. 1, the device 22 includes a heat exchanger 22a configured to exchange heat with the blood, a gaseous micro-emboli (GME) removal module 22b configured to remove gaseous micro-emboli from the blood, and a gas exchanger 22c, also referred to as an oxygenator 22c, configured to exchange O2 and CO2 with the blood.

In other embodiments, the device 22 includes different functional components or modules. In some embodiments, the device 22 includes only an oxygenator 22c configured to exchange O2 and CO2 with the blood. In some embodiments, the device 22 includes only a heat exchanger 22a configured to exchange heat with the blood and an oxygenator 22c configured to exchange O2 and CO2 with the blood. In some embodiments, the device 22 includes only a GME removal module 22b configured to remove gaseous micro-emboli from the blood and an oxygenator 22c configured to exchange O2 and CO2 with the blood. In some embodiments, the system 20 includes other components, such as other heat exchangers, GME removers, and gas exchangers. In some embodiments, these components or modules are structurally integrated into the device 22. In other embodiments, these components or modules are exchangeable, such that various modules may be easily added or removed from the device 22.

The system 20 includes the device 22, a processing unit 24, and a blood reservoir 26. In embodiments, the processing unit 24 may be a heart lung machine (HLM) or part of an HLM, as used in cardiopulmonary bypass surgery. Also, embodiments of the subject matter described herein may be implemented within the context of any number of different processing units, blood reservoirs, heat exchangers, GME removers, and oxygenators.

The processing unit 24 includes components, such as pumps, one or more controller assemblies including computing devices, one or more peripheral display devices, and one or more user interfaces, for providing the functions of the system 20. The functions of the system 20 include receiving blood from a patient 28, providing the blood to the blood reservoir 26, and supplying the blood from the blood reservoir 26 to the blood conditioning device 22. After being conditioned in the device 22, the blood can be returned to the patient 28.

The processing unit 24 is operatively coupled to the patient 28 and to the extracorporeal blood circulation system. Blood is taken from the patient 28 by means of a venous blood tubing line 30 and received by the blood reservoir 26. Besides this, blood may be aspirated into the reservoir 26 by means of one or more suckers from the operating field and be otherwise received into the blood reservoir 26 from the patient 28. In embodiments, the blood reservoir 26 includes a filter for filtering the blood received from the patient 28 and the operating field, prior to passing the blood to the device 22.

The device 22 is fluidly coupled to the blood reservoir 26 to receive the blood from the blood reservoir 26. A roller, or centrifugal blood pump 32 that is controlled by processing unit 24 may be interposed between the blood reservoir 26 and the blood conditioning device 22. The pump 32 is fluidly coupled to the blood reservoir by blood tubing line 34 and fluidly coupled to the device 22 by blood tubing line 36. The pump 32 is used for pumping blood from the outlet port of the blood reservoir 26 through the blood tubing line 34 and to the inlet port of the device 22 through the blood tubing line 36.

The device 22 is coupled to a heater/cooler 38 that provides a fluid, such as water, at a controlled temperature to the heat exchanger 22a through inlet fluid tubing line 40 for heating/cooling the blood. The heater/cooler 38 receives returning fluid from the blood conditioning device 22 through the outlet fluid tubing line 42. In embodiments, the heater/cooler 38 is controlled by the processing unit 24.

The device 22 is further fluidly coupled to vacuum source 44 by fluid tubing line 46 for applying a vacuum through the fluid tubing line 46 to the GME removal module 22b. Alternatively, atmospheric pressure may be provided to the GME removal module 22b, such as by keeping the fluid tubing line 46 open to the atmosphere. In embodiments, the fluid tubing line 46 provides from atmospheric pressure to a vacuum of -400mmHG to the GME removal module 22b for removing GME from the blood. In embodiments, the vacuum source 44 is controlled by the processing unit 24.

The device 22 is further fluidly coupled to a gas mixture module or gas blender 48 by inlet fluid tubing line 50. The gas blender 48 provides oxygenation gases to the oxygenator 22c through inlet fluid tubing line 50 to oxygenate the processed blood. The gas outlet 52 of the device 22 is kept fully open to the atmosphere, such that given the pressure losses in the oxygenator gas, oxygenation takes place, as usually happens with all existing oxygenators, with the oxygenation gases at pressure values slightly over or close to atmospheric pressure values. In embodiments, the gas blender 48 is controlled by the processing unit 24.

The device 22 receives the blood from the reservoir 26 through the blood tubing line 36 and processes the blood by exchanging heat with the blood in the heat exchanger 22a, removing GME from the blood in the GME removal module 22b, and oxygenating the blood in the oxygenator 22c. The device 22 returns the processed blood to the patient 28 through blood tubing line 54. In embodiments, the device 22 is operatively coupled to the processing unit 24, which controls the return of the temperature-controlled, degassed, and oxygenated blood to the patient 28 through the blood tubing line 54, such as an arterial line.

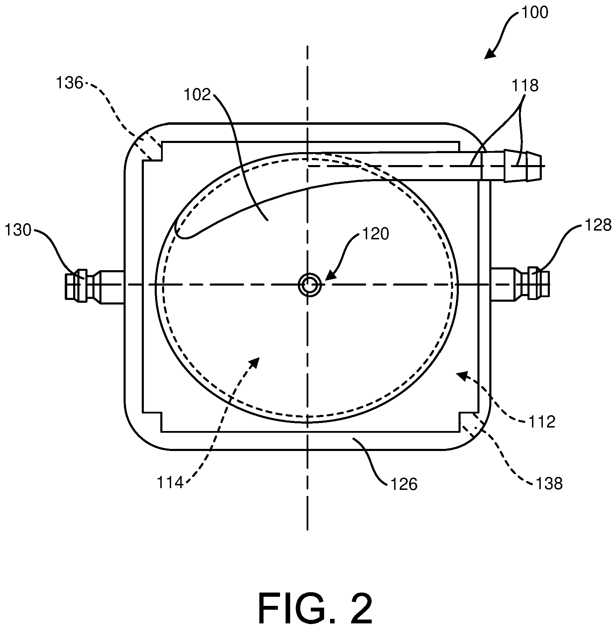

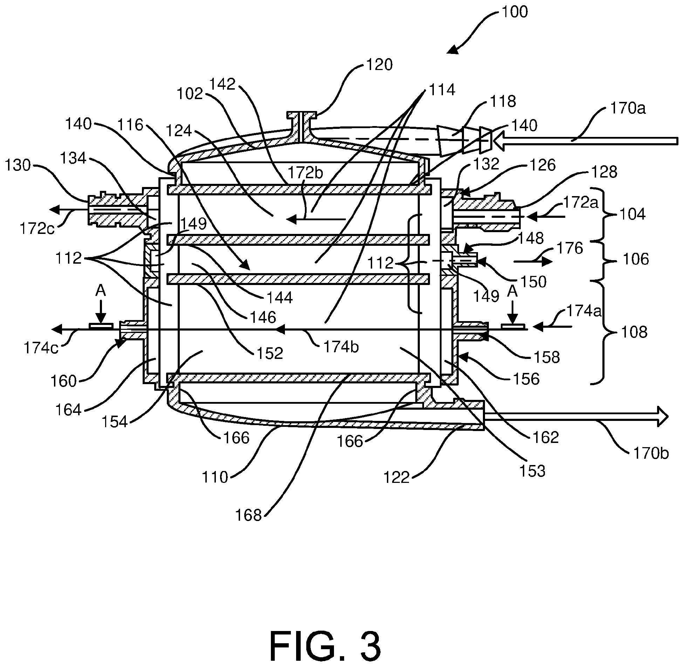

Figs. 2 and 3 are diagrams illustrating a modular blood conditioning device 100, in accordance with embodiments of the subject matter of the disclosure. Fig. 2 is a diagram illustrating a top view of the modular blood conditioning device 100, in accordance with embodiments of the subject matter of the disclosure. Fig. 3 is a diagram illustrating a longitudinal section view of the modular blood conditioning device 100, in accordance with embodiments of the subject matter of the disclosure. In embodiments, the blood conditioning device 100 is like the blood conditioning device 22 (shown in Fig. 1). In embodiments, the device 100 can be used in place of device 22 in system 20.

Generally, the device 100 includes a blood inlet cap 102, a heat exchanger module 104, a GME removal module 106, a gas exchanger or oxygenator module 108, and a blood outlet cap 110. Each of the modules, including the heat exchanger module 104, the GME removal module 106, and the gas exchanger module 108, includes a pile of stacked single hollow fiber mat layers that are alternatively stacked at 90° angles. That is, each of the hollow fiber mat layers includes a layer of hollow fibers that are parallel to one another and define the hollow fiber direction for that layer. These layers are alternatively stacked with 90° angle hollow fiber directions. Also, the characteristics of the hollow fiber mat layers in one module may be and often are different from the characteristics of the hollow fiber mat layers in the other modules.

The three modules are potted with a potting material (resin) in a single step to form a single potting material body 112 that defines a single, unified, cylindrical blood compartment 114 through the three modules. End portions of the hollow fibers of the hollow fiber mat layers are embedded in the potting material body 112 and the internal surface of the potting material body 112 circumscribes and defines the blood compartment 114. The blood compartment 114 provides a blood flow path through the three modules, where the blood flows on the outside of the hollow fibers of the hollow fiber mat layers of the three modules. This results in the device 100 having a cylindrical internal shape for the blood to flow through from the blood inlet cap 102 to the blood outlet cap 110.

Further, during manufacturing the ends of the hollow fibers of the hollow fiber mat layers of the three modules are embedded in the potting material that eventually becomes the potting material body 112. This leads to the potting material having an outside squared cross-section that is cut away on the external surfaces to expose the inner lumens of the hollow fibers of the hollow fiber mat layers and provide access to the inner lumens of the hollow fibers from outside the potting material body 112. Each of the three modules is further fitted with an external housing that includes one or more connectors to provide appropriate fluids/gases to the device.

Thus, device 100 includes stacked hollow fiber mat layers such that the blood flows outside the hollow fibers from the blood inlet cap 102 to the blood outlet cap 110 in a direction that is transverse to the stacked hollow fiber mat layers. This results in a shorter blood flow path than in a cylindrically wound blood conditioner and the resulting pressure gradient is lower, which reduces or prevents mechanical damage to blood cells. Also, the blood compartment 114 is cylindrical such that the blood flow path does not include dead angles where blood can stagnate and start to clot. In addition, the potting material (resin) is applied to all three modules in one step to provide a single, unified, blood compartment 114, which reduces the complexity and cost of manufacturing the device 100.

As illustrated in Figs. 2 and 3, the blood inlet cap 102 includes a blood inlet port 118 through which blood enters the device 100 and a purging line port 120 through which air can be purged from the device 100. In embodiments, as illustrated, the blood inlet port 118 is situated tangentially on the blood inlet cap 102 and the purging line port 120 is situated at the center of the blood inlet cap 102. In other embodiments, the blood inlet port 118 can be situated at the center of the blood inlet cap 102.

The blood enters the device 100 through the blood inlet port 118 as shown by arrow 170a and flows in a circular motion around the blood inlet cap 102 and into the cylindrical blood compartment 114. In embodiments, as illustrated, the blood flows through the blood inlet port 118 of the blood inlet cap 102 and then through the heat exchanger module 104, the GME removal module 106, and the gas exchanger module 108 before exiting the device 100 through the blood outlet port 122 of the blood outlet cap 110 as shown by arrow 170b.

The first module in the cascade of three modules to be crossed by the blood is the heat exchanger module 104. The heat exchanger module 104 includes heat exchanger hollow fibers in stacked heat exchanger hollow fiber mat layers 124. The heat exchanger hollow fibers at 124 are configured to receive a heat exchanger fluid, such as water, for exchanging heat with the blood.

The heat exchanger module 104 includes a heat exchanger housing 126 that is external to the potting material body 112 and that includes a fluid inlet port 128 and a fluid outlet port 130. The heat exchanger housing 126 is sealed to the potting material body 112 to prevent heat exchanger fluid from leaking out and divided into two inner chambers, an inlet fluid chamber 132 and an outlet fluid chamber 134. The heat exchanger housing 126 is divided into the two inner chambers, by two diametrically opposed longitudinal spacers 136 and 138 (shown in Fig. 2). The inlet fluid chamber 132 puts the fluid inlet port 128 in fluid communication with one side or end of the heat exchanger hollow fibers at 124. The outlet fluid chamber 134 puts the fluid outlet port 130 in fluid communication with the opposite side or end of the heat exchanger fibers at 124. The heat exchanger fluid (e.g., water) flows from the inlet fluid port 128 and the inlet fluid chamber 132 into the inner lumens of the heat exchanger hollow fibers at 124 and is collected at the outlet fluid chamber 134 to exit the device 100 through the fluid outlet port 130, before being circulated back to the heater/cooler equipment, such as heater/cooler 38 (shown in Fig. 1). The heat exchanger housing 126 is the first of three module housings, where each of them is sealed to the external surface of the potting material body 112 and encloses one of the three modules.

Also, in embodiments, the device 100 includes blood inlet cap supports 140 embedded in the potting material body 112 and extending above the heat exchanger hollow fiber mat layers 124. The blood inlet cap 102 is attached to and supported by the blood inlet cap supports 140. In embodiments, the device 100 includes an inlet cap spacer 142 situated on the heat exchanger hollow fiber mat layers 124 and embedded in the potting material body 112. In embodiments, the device 100 includes the blood inlet cap supports 140 attached to or on the inlet cap spacer 142.

In addition, in embodiments, the device 100 includes a module spacer 144 between the heat exchanger module 104 and the GME removal module 106. The module spacer 144 is embedded into the potting material body 112. In embodiments, the module spacer 144 is made of plastic.

The second module in the cascade of three modules to be crossed by the blood is the GME removal module 106. The GME removal module 106 includes micro-porous hollow fibers in stacked micro-porous hollow fiber mat layers 146. The GME micro-porous hollow fibers at 146 are different from the heat exchanger hollow fibers at 124, in that the micro-porous hollow fibers at 146 are permeable to gas and impermeable to liquids, while the heat exchanger hollow fibers at 124 are permeable only to energy or heat. Also, the heat exchanger hollow fiber mats 124 and the micro-porous hollow fiber mats 146 may differ in fiber linear density, dimensions, and number of warp yarns.

In the GME removal module 106, atmospheric pressure or sub-atmospheric pressure (a vacuum) is applied to the inner lumens of the micro-porous hollow fibers at 146 to pull GME from the blood through the walls of the micro-porous hollow fibers at 146. This removes the GME form the blood, where the GME needs to be removed from the blood before returning the blood to the patient in order to avoid embolization.

The GME removal module 106 includes a GME removal module housing 148 that includes a fluid outlet 150 in fluid communication with at least one side of the micro-porous hollow fibers at 146. The GME removal module housing 148 is sealed to the potting material body 112 and provides atmospheric or sub-atmospheric pressures received at the fluid outlet 150 to the micro-porous hollow fibers at 146. In embodiments, the fluid outlet 150 is in fluid communication with an inner chamber 149 that is in fluid communication with all sides of the micro-porous hollow fibers at 146.

The third module in the cascade of three modules to be crossed by the blood is the gas exchanger or oxygenator module 108. In embodiments, the device 100 includes a module spacer 152 situated between the GME removal module 106 and the gas exchanger module 108. The module spacer 152 includes, on the GME removal module 106 side, a filter screen 116 whose function is to retain gaseous micro-emboli in the GME removal module 106 to facilitate their removal. The module spacer 152 and the filter screen 116 are embedded into the potting material body 112. In embodiments, the module spacer 152 and/or the filter screen 116 are made of plastic.

The gas exchanger module 108 includes gas exchanger hollow fibers in stacked gas exchanger hollow fiber mat layers 154. The gas exchanger hollow fibers at 154 are configured to receive a gas mixture and exchange gas with the blood. The gas exchanger hollow fibers at 154 are different from the heat exchanger hollow fibers at 124, in that the gas exchanger hollow fibers at 154 are permeable to gas and impermeable to liquids, while the heat exchanger hollow fibers at 124 are permeable only to energy or heat. Also, the heat exchanger hollow fiber mat layers 124, the micro-porous hollow fiber mat layers 146, and the gas exchanger hollow fiber mat layers 154 may each differ in fiber linear density, dimensions, and number of warp yarns.

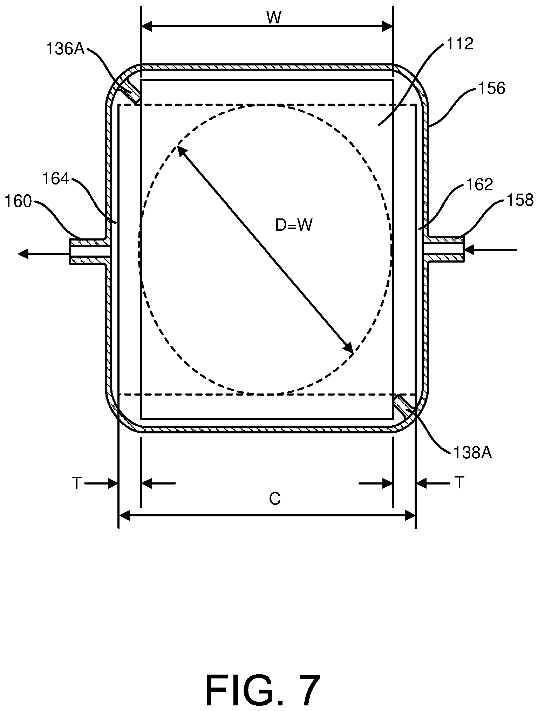

The gas exchanger module 108 includes a gas exchanger housing 156 that includes opposed gas exchanger fluid connectors, a gas inlet port 158 and a gas outlet port 160. The gas exchanger housing 156 is sealed to the potting material body 112 to prevent the gas mixture from leaking out. The gas exchanger housing 156 is divided into two inner chambers, an inlet gas chamber 162 and an outlet gas chamber 164, by the two diametrically opposed longitudinal spacers 136A and 138A (shown in Fig. 7). The inlet gas chamber 162 puts the gas inlet port 158 in fluid communication with one side or end of the gas exchanger hollow fibers at 153. The outlet gas chamber 164 puts the gas outlet port 160 in fluid communication with the opposite side or end of the gas exchanger fibers at 154. In this way, the gas mixture flows from the gas inlet port 158 and the gas inlet chamber 162 into the inner lumens of the gas exchanger hollow fibers at 154 and is collected at the gas outlet chamber 164 to exit the device 100 through the gas outlet port 160.

In embodiments, the device 100 includes one or more blood outlet cap supports 166 embedded in the potting material body 112. Also, in embodiments, the device 100 includes an arterial blood filter 168 situated under the gas exchanger module 108 and between the gas exchanger module 108 and the blood outlet cap 110. The arterial blood filter 168 is obtained with one or more screen layers of suitable size, such as 20 to 40 microns, and embedded in the potting material body 112. In operation, the blood flows through the gas exchanger module 108 and the arterial blood filter 168 into the blood outlet cap 110 and out of the device 100 through the blood outlet port 122.

In other embodiments, the device 100 may include only one or two of: the heat exchanger module 104; the GME removal module 106; and the gas exchanger module 108. These different embodiments may be used in different applications. In some embodiments, such as extracorporeal membrane oxygenation (ECMO) or extracorporeal life support (ECLS), the heat exchanger module 106 may not be needed. In some embodiments, such as for cardiac surgery, the device 100 can include at least the heat exchanger module 104 and the oxygenator module 108. In some embodiments, the GME removal module 106 is included for extra safety by eliminating or reducing the GME in the blood.

The flow of heat exchanger fluid, such as water, and gas exchanger fluid, such as oxygen, through the hollow fiber mat layers is shown by arrows in Fig. 3. As shown by arrows 172a - 172c, the heat exchanger fluid flows through the heat exchanger module 104 from the fluid inlet port 128 at arrow 172a, through the heat exchanger hollow fibers at 124 at arrow 172b, and through the fluid outlet port 130 at arrow 172c. As shown by arrows 174a - 174c, the gas exchanger fluid flows through the gas exchanger module 108 from the gas inlet port 158 at arrow 174a, through the gas exchanger hollow fibers at 154 at arrow 174b, and through the gas outlet port 160 at arrow 174c. As shown by arrow 176, the GME are removed from the GME removal module 106 at arrow 176. In various alternative embodiments, the device 100 can be configured such that the heat exchanger fluid and the gas exchanger fluid flow through the device 100 in opposing directions.

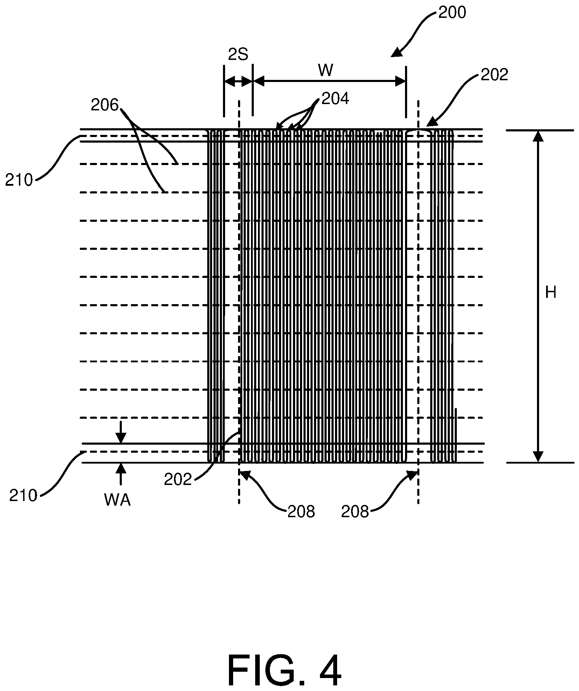

Figs. 4-7 are diagrams illustrating the construction of the device 100, in accordance with embodiments of the subject matter of the disclosure. Each of the modules, including the heat exchanger module 104, the GME removal module 106, and the gas exchanger module 108, includes a pile of stacked single hollow fiber mat layers that are alternatively stacked at 90° angles. That is, each of the hollow fiber mat layers includes a layer of hollow fibers that are parallel to one another and define the hollow fiber direction for that layer. These layers are alternatively stacked with 90° angle hollow fiber directions. Also, the characteristics of the hollow fibers in one module may be different from the characteristics of the hollow fibers in the other modules. Construction of one hollow fiber mat layer is described below, where the same construction technique can be used for each of the hollow fiber mat layers in the heat exchanger module 104, the GME removal module 106, and the gas exchanger module 108.

Fig. 4 is a diagram illustrating the construction of a hollow fiber mat layer 200 used in the device 100, in accordance with embodiments of the subject matter of the disclosure. The hollow fiber mat layer 200 is to be stacked in one of the modules and has a rectangular shape, where H corresponds to the height and W to the width, with the height H greater than the width W. In embodiments, each of the hollow fiber mat layers has the same height H and width W dimensions for all three of the modules.

The hollow fiber mat layer 200 is obtained by knitting a mat or weave that has a discontinued weft and cutting the weave into pieces at spaces 202 where the weft is missing. The hollow fiber mat layer 200 includes hollow fibers 204 as the weft and yarn 206 as the warp. A single layer of hollow fibers 204 is knitted into a rectangle having the height H of the hollow fibers 204 and the width W. The weft is discontinued, such that the spaces 202 having width 2S are left between the hollow fiber mat layer 200 and other mat layers that may be knit on each side of the hollow fiber mat layer 200. The yarn 206 is cut at 208 in the middle of the spaces 202 (1S from the hollow fiber mat layer 200) on each side of the hollow fiber mat layer 200 to remove the hollow fiber mat layer 200. In embodiments, an adhesive layer 210 having a width WA is attached to the top and to the bottom of the hollow fibers 204. In embodiments, hollow fiber mat layers may be knit with different materials, have different fiber dimensions, have different linear density, and have a different number of warp yarns for each of the modules.

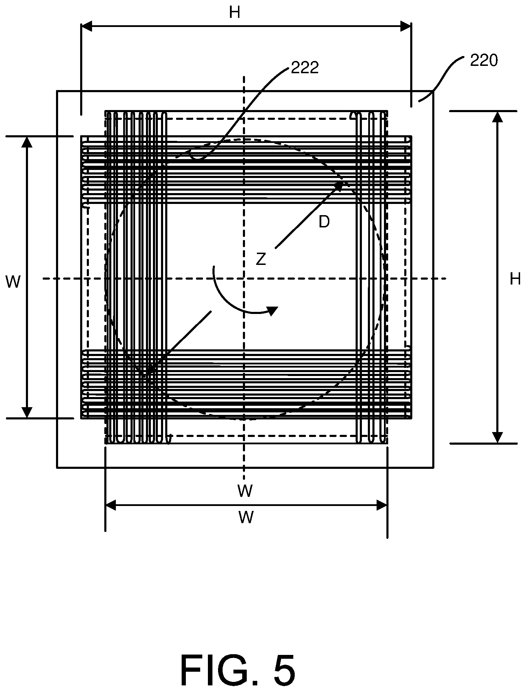

Fig. 5 is a top view diagram illustrating the components of the device 100, including the modules 104, 106, and 108, piled into a potting mold 220 and ready for potting, in accordance with embodiments of the subject matter of the disclosure. For each of the modules 104, 106, and 108, hollow fiber mat layers, such as hollow fiber mat layer 200, are stacked by alternatively crossing them at 90°, one on top of the other, according to the number of hollow fiber mat layers prescribed for the module. Each of the hollow fiber mat layers has a height H and a width W. The modules are situated in the potting mold 220, which keeps them aligned. In embodiments, different modules can have a different number of hollow fiber mat layers.

In embodiments, the components of the device 100 are piled into the potting mold 220 in the following order. First, the blood outlet cap supports 166 (shown in Fig. 3) are situated or inserted into the potting mold 220 followed by the arterial blood filter 168. Next, the gas exchanger module 108 is piled in the potting mold 220 followed by the module spacer 152 and the GME removal module 106. Next, the module spacer 144 is piled into the potting mold 220 followed by the heat exchanger module 104, the inlet cap spacer 142, and the blood inlet cap supports 140.

With all of these components loaded into the potting mold 220, potting is done in one step by spinning the potting mold 220 around the vertical axis Z at a certain speed and introducing the liquid potting material (resin) into the potting mold 220. The liquid potting material spreads to the outside of the potting mold 220 due to centrifugal forces and embeds the components of the device 100 in the potting material. At the end of the potting phase, the central portion of the potting material pack, which is the cylindrical blood compartment 114, is an open space occupied by the stacked horizontal hollow fiber mat layers of the modules and circumscribed by the inner surface 222 of the potting material body 112. The outer surface of the potting material has the same shape of the potting mold 220, that can be removed after resin curing, resulting in a potted body of the device 100. In embodiments, the potting mold 220 includes silicon rubber.

The diameter D (shown in Fig. 5) of the inner surface of the potting material body 112 can be calculated to be equal to the width W of the hollow fiber mat layers. If it is, the open space in the blood compartment 114 is evenly occupied by 90° crossed hollow fibers, such that no preferential channels are created in the blood flow path and no hollow fibers are completely embedded in the potting material and cut off from contact with the blood. If it is not, the exchange efficiencies of the device 100 may be less than optimal.

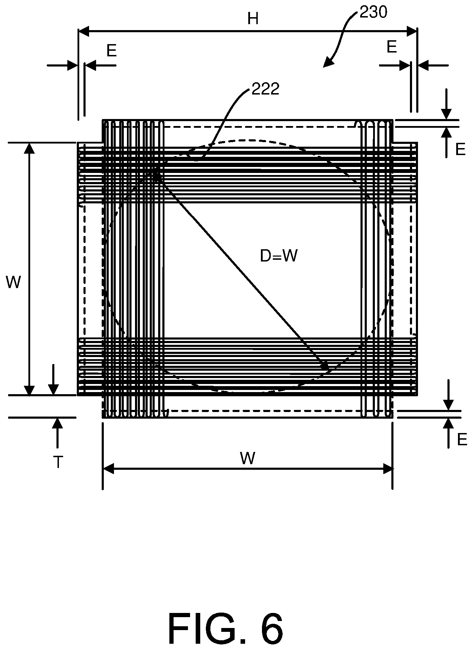

Fig. 6 is a diagram illustrating a cross sectional view of the potted body 230 of the device 100, in accordance with embodiments of the subject matter of the disclosure. The potted body 230 has the shape of the potting mold 220.

After removing the potting mold 220, the potted body 230 is ready for being cut to form the outside of the potting material body 112. In this phase of the construction, access to the inner lumens of the hollow fibers is obtained by removing the potting material (resin) that covers the ends of the hollow fibers. As illustrated, a thin slice E of the potting material is removed from each outer side of the potted body 230, where E must be large enough for exposing the ends of the hollow fibers on all sides of the potted body 230. This results in the cross-section of the cut potted body 230 being inscribed in a square having a side C=W+2T=H-2E, where T is the potting material minimum thickness.

Fig. 7 is a cross-section of the device 100 along the line A-A in Fig. 3, corresponding to a middle height line of the gas exchanger module 108, in accordance with embodiments of the subject matter of the disclosure. The gas exchanger housing 156, including the gas inlet port 158 and the gas outlet port 160, is situated around the cut potted body 230, which is now the potting material body 112, and sealed to the potting material body 112. The diametrically opposed longitudinal spacers 136A and 138A are introduced with radial interference at two diametrically opposed corners to create the inlet gas chamber 162 and the outlet gas chamber 164. The same or a similar construction is repeated for the heat exchanger module 104 and the GME removal module 106, where the GME removal module housing 148 has only one fluid outlet 150 and one corresponding inner chamber 149. In embodiments, the opposed longitudinal spacers 136A and 138A are made of rubber.

In assembly of the device 100, the heat exchanger housing 126, GME removal module housing 148, and gas exchanger housing 156 are attached to the cut potted body 230, which is now the potting material body 112. The blood inlet cap 102 is attached to the blood inlet cap supports 140 and sealed adjacent the inlet cap spacer 142, which is situated on the heat exchanger hollow fiber mat layers 124 and embedded in the potting material body 112. The blood inlet cap 102 is shaped to impart a spiral flow direction to the entering blood, see Fig. 2, such that if air bubbles are present, the induced centrifugal force moves them towards the central purging port 120 where they are easily removed by the purging line. In embodiments, this is useful during the device 100 initial priming with liquid.

The blood outlet cap 110 is attached to the blood outlet cap supports 166 that are embedded in the potting material body 112. The blood outlet cap 110 is sealed adjacent the arterial blood filter 168 situated under the gas exchanger module 108 and between the gas exchanger module 108 and the blood outlet cap 110.

In embodiments, laser welding can be used to seal one or more of the blood inlet cap 102, the blood outlet cap 110, the heat exchanger housing 126, the GME removal module housing 148, and the gas exchanger housing 156 to the underlying potting material body 112 to form the device 100.

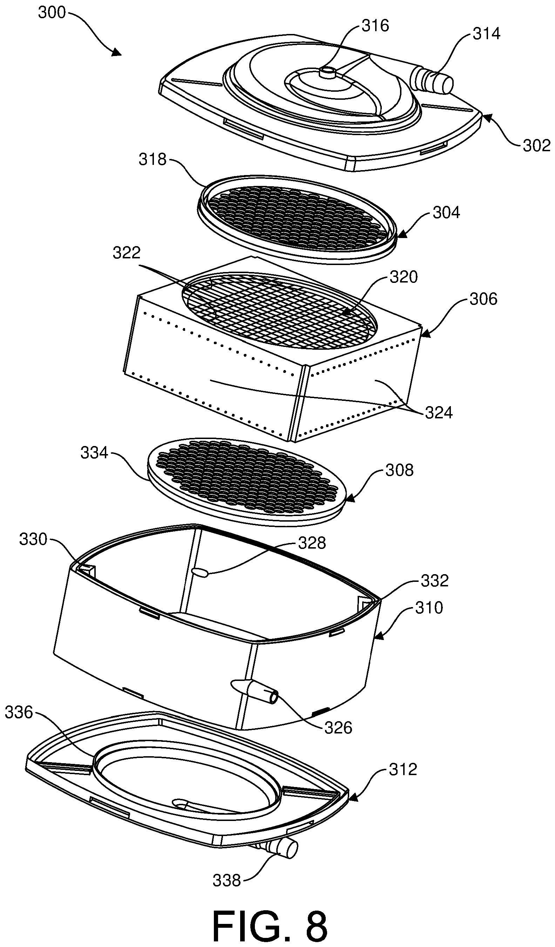

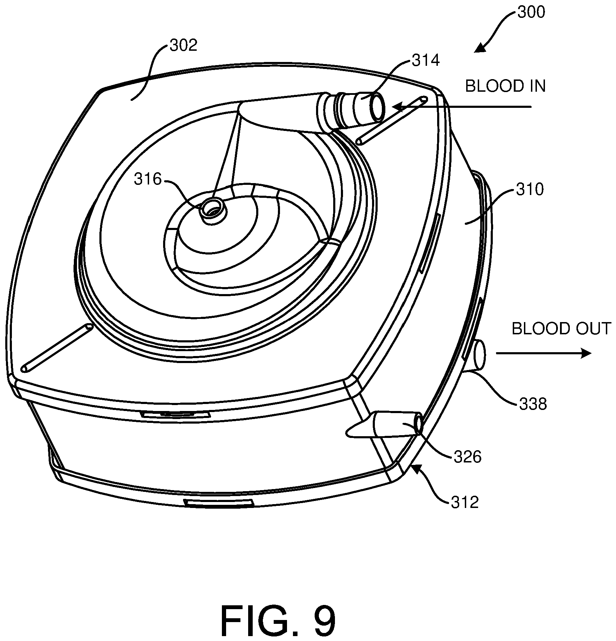

Figs. 8 and 9 are diagrams illustrating a blood conditioning device 300 including only a gas exchanger module, such as gas exchanger module 108, in accordance with embodiments of the subject matter of the disclosure. Fig. 8 is a diagram illustrating an exploded view of the device 300, in accordance with embodiments of the subject matter of the disclosure.

The device 300 includes a blood inlet cap 302, a blood inlet cap spacer 304, a cut potted body 306, an arterial blood filter 308, a gas exchanger housing 310, and a blood outlet cap 312. The blood enters the blood inlet cap 302 and flows through the blood inlet cap spacer 304, the cut potted body 306, and the arterial blood filter 308, before exiting through the blood outlet cap 312.

The blood inlet cap 302 includes a blood inlet port 314 through which blood enters the device 300 and a purging line port 316 through which air can be purged from the device 300. In embodiments, as illustrated in Figs.8 and 9, the blood inlet port 314 is situated tangentially on the blood inlet cap 302 and the purging line port 316 is situated at the center of the blood inlet cap 302. In other embodiments, the blood inlet port 314 can be situated at the center of the blood inlet cap 302.

In embodiments as illustrated in Fig. 8, the blood inlet cap spacer 304 may be embedded in the potting material of the cut potted body 306, or the blood inlet cap spacer 304 may be a separate unit that is sealed, such as by adhesives or laser welding, onto the potted body 306 to prevent leakage of the blood. Also, the blood inlet cap 302 is sealed to and supported by blood inlet cap supports 318 that are part of the blood inlet cap spacer 304. In embodiments, the blood inlet cap 302 is sealed, such as by adhesives or laser welding, to the blood inlet cap supports 318 to prevent leakage of the blood.

The cut potted body 306 includes gas exchanger hollow fiber mat layers 320 situated in a cylindrical blood compartment 322. The cut potted body 306 is like the cut potted body 230 in that it has been constructed using the construction techniques used to construct the cut potted body 230. The sides of the cut potted body 306 include exposed inner lumens of the gas exchanger hollow fibers at 324 in the gas exchanger hollow fiber mat layers 320.

The gas exchanger housing 310 includes opposed gas exchanger fluid connectors, a gas inlet port 326 and a gas outlet port 328. The gas exchanger housing 310 is sealed to the cut potted body 306 to prevent gas from leaking out. The gas exchanger housing 310 is divided into an inlet gas chamber and an outlet gas chamber by diametrically opposed longitudinal spacers 330 and 332 that are inserted in diametrically opposed corners of the gas exchanger housing 310. These longitudinal spacers 330 and 332 are like the longitudinal spacers 136A and 138A (shown in Fig. 7). In embodiments, the longitudinal spacers are made from or at least include rubber.

The inlet gas chamber puts the gas inlet port 326 in fluid communication with one side of the inner lumens of the gas exchanger hollow fibers at 324. The outlet gas chamber puts the gas outlet port 328 in fluid communication with the opposite side of the inner lumens of the gas exchanger hollow fibers. In this way, gas flows from the gas inlet port 326 through the inner lumens of the gas exchanger hollow fibers and exits the device 300 through the gas outlet port 328.

In embodiments as illustrated in Fig. 8, the arterial blood filter 308 may be embedded in the potting material of the potted body 306, or the arterial blood filter 308 may be a separate unit that is sealed, such as by adhesives or laser welding, onto the potted body 306 to prevent leakage of the blood.

The arterial blood filter 308 includes blood outlet cap supports 334 that are attached to a corresponding ring 336 on the blood outlet cap 312. The ring 336 on the blood outlet cap 312 is sealed, such as by adhesives or laser welding, to the blood outlet cap supports 334 to prevent leakage of the blood. The gas exchanger housing 310 is further attached and sealed to the blood inlet cap 302 and to the blood outlet cap 312.

In operation, a gas mixture is provided to the gas inlet port 326 to flow through the inner lumens of the gas exchanger hollow fiber mat layers 320 and out the gas outlet port 328. The blood enters the device 300 through the blood inlet port 314 and flows in a circular motion around the blood inlet cap 302 and through the blood inlet cap spacer 304 and into the cylindrical blood compartment 322 of the potted body 306. Next, the blood flows through the hollow fiber mat layers 320 and then through the arterial blood filter 308 and into the blood outlet cap 312. The blood exits the device 300 through the blood outlet port 338 of the blood outlet cap 312.

Fig. 9 is a diagram illustrating an assembled view of the device 300, in accordance with embodiments of the subject matter of the disclosure. The device 300 includes the blood inlet cap 302 with the blood inlet port 314 and the blood outlet cap 312 with the blood outlet port 338. The gas exchanger housing 310 includes the gas inlet port 326 and the gas outlet port 328 (not shown in Fig. 9).

In operation, a gas mixture is provided to the gas inlet port 326 for flowing though the gas exchanger hollow fiber mats 320 and the blood is provided at the blood inlet port 314 to flow through the device 300 before exiting through the blood outlet port 338 of the blood outlet cap 312.

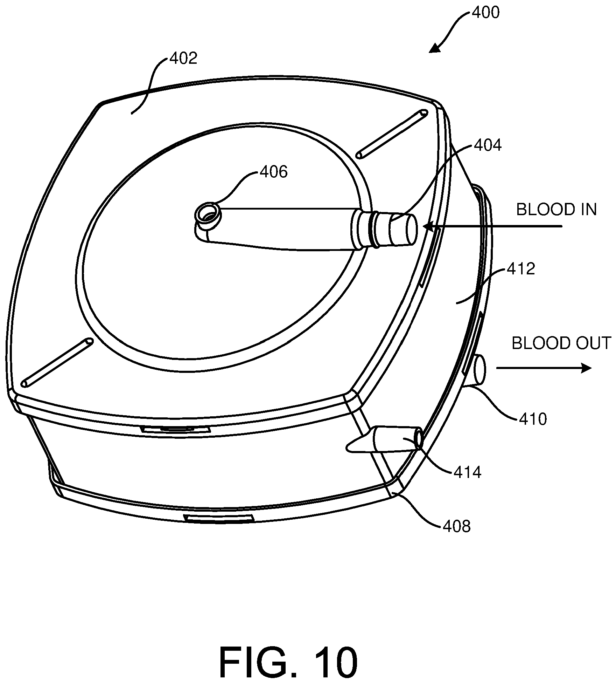

Fig. 10 is a diagram illustrating an assembled view of another device 400, in accordance with embodiments of the subject matter of the disclosure. The device 400 is like the device 300 except the blood inlet cap 402 has a centrally located blood inlet port 404 instead of a tangentially located blood inlet port. The device further includes a centrally located purging line port 406, a blood outlet cap 408 with a blood outlet port 410, and a gas exchanger housing 412 with a gas inlet port 414 and a gas outlet port (not shown).

In operation, a gas mixture is provided to the gas inlet port 414 for flowing though the gas exchanger hollow fiber mats and the blood is provided at the blood inlet port 404 to flow through the device 400 before exiting through the blood outlet port 410 of the blood outlet cap 408.

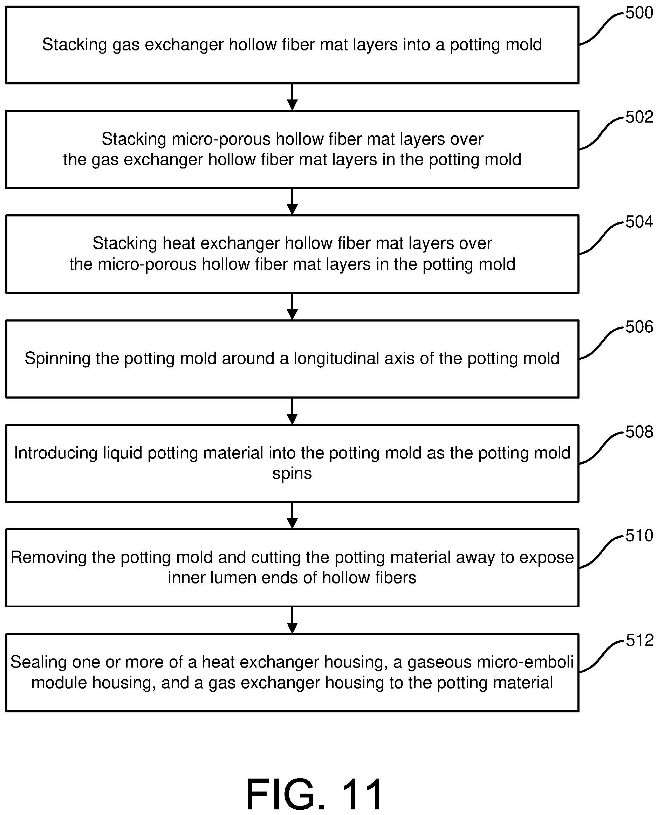

Fig. 11 is a flow chart diagram illustrating a method of manufacturing a device for conditioning blood, in accordance with embodiments of the subject matter of the disclosure.

At 500, the method includes stacking gas exchanger hollow fiber mat layers into a potting mold. In embodiments, the method further includes one or more of placing a blood outlet cap support in the potting mold and placing an arterial blood filter in the potting mold and then stacking the gas exchanger hollow fiber mat layers into the potting mold.

At 502, the method includes stacking micro-porous hollow fiber mat layers over the gas exchanger hollow fiber mat layers in the potting mold. In embodiments, the method includes situating a first spacer between the gas exchanger hollow fiber mat layers and the micro-porous hollow fiber mat layers.

At 504 the method includes stacking heat exchanger hollow fiber mat layers over the micro-porous hollow fiber mat layers in the potting mold. In embodiments, the method includes situating a second spacer between the micro-porous hollow fiber mat layers and the heat exchanger hollow fiber mat layers and/or a blood inlet cap support on the heat exchanger hollow fiber mat layers, prior to spinning the potting mold around the longitudinal axis of the potting mold and introducing liquid potting material into the potting mold.

At 506, the method includes spinning the potting mold around a longitudinal axis of the potting mold, and at 508, the method includes introducing liquid potting material into the potting mold as the potting mold spins to embed the gas exchanger hollow fiber mat layers and the micro-porous hollow fiber mat layers and the heat exchanger hollow fiber mat layers in the potting material and create a blood compartment that extends through the gas exchanger hollow fiber mat layers and the micro-porous hollow fiber mat layers and the heat exchanger hollow fiber mat layers. Where, in embodiments, introducing the liquid potting material into the potting mold as the potting mold spins creates a cylindrical blood compartment defined by an inner diameter of the potting material.

At 510, after potting material curing, the method includes removing the potting mold and cutting the potting material away to expose inner lumen ends of heat exchanger hollow fibers in the heat exchanger hollow fiber mat layers and inner lumen ends of micro-porous hollow fibers in the micro-porous hollow fiber mat layers and inner lumen ends of gas exchanger hollow fibers in the gas exchanger hollow fiber mat layer.

At 512, the method includes sealing one or more of a heat exchanger housing to the potting material, a gaseous micro-emboli module housing to the potting material, and a gas exchanger housing to the potting material. Also, in embodiments, the method further includes attaching a blood inlet cap and attaching a blood outlet cap.

In embodiments, the method can further include creating one or more of knitting a discontinuous weft of a single layer of gas exchanger hollow fibers and cutting the discontinuous weft where the single layer of gas exchanger hollow fibers is missing to provide a gas exchanger hollow fiber mat layer that is stacked into the potting mold; knitting a discontinuous weft of a single layer of micro-porous hollow fibers and cutting the discontinuous weft where the single layer of micro-porous hollow fibers is missing to provide a micro-porous hollow fiber mat layer that is stacked into the potting mold; and knitting a discontinuous weft of a single layer of heat exchanger hollow fibers and cutting the discontinuous weft where the single layer of heat exchanger hollow fibers is missing to provide a heat exchanger hollow fiber mat layer that is stacked into the potting mold.

## Claims

1. A device (100) for conditioning blood comprising:
a heat exchanger module (104) including a heat exchanger fiber layer (124) including heat exchanger fibers configured to receive a heat exchanger fluid and exchange heat with the blood;
a gaseous micro-emboli removal module (106) including a micro-porous fiber layer (146) including micro-porous fibers configured to receive atmospheric or sub- atmospheric pressures such that at least some gaseous micro-emboli are drawn from the blood through the micro-porous fibers;
a gas exchanger module (108) including a gas exchanger fiber layer (154) including gas exchanger fibers configured to receive a gas mixture and exchange gas with the blood; and
a potting material body (112) that embeds the heat exchanger fibers, the micro-porous fibers and the gas exchanger fibers and defines a blood compartment (114) that extends through the heat exchanger module (104), the gaseous micro-emboli removal module (106), and the gas exchanger module (108);
**characterized by** at least one of:
the heat exchanger module (104) including stacked heat exchanger hollow fiber mat layers (124), wherein the heat exchanger hollow fiber mat layers (124) are alternatively orthogonally angled from one another;
the gaseous micro-emboli removal module (106) including stacked micro-porous hollow fiber mat layers (146), wherein the micro-porous hollow fiber mat layers (146) are alternatively orthogonally angled from one another; and
the gas exchanger module (108) including stacked gas exchanger hollow fiber mat layers (154), wherein the gas exchanger hollow fiber mat layers (154) are alternatively orthogonally angled from one another.

2. The device of claim 1, comprising a heat exchanger housing (126) sealed to the potting material body (112) and having an inlet (128) and an outlet (130), wherein the heat exchanger housing (126) provides a first chamber (132) in fluid communication with the inlet (128) and inner lumens of the heat exchanger fibers on one side of the heat exchanger fibers and a second chamber (134) in fluid communication with the outlet (130) and the inner lumens of the heat exchanger fibers on the opposite side of the heat exchanger fibers.

3. The device of claim 1, comprising a gaseous micro-emboli removal module housing (148) sealed to the potting material body (112) and having a fluid outlet (150) in fluid communication with at least inner lumens of the micro-porous fibers on one side of the micro-porous fibers.

4. The device of claim 1, comprising a gas exchanger housing (156) sealed to the potting material body (112) and having a gas inlet (158) and a gas outlet (160), wherein the gas exchanger housing (156) provides a first chamber (162) in fluid communication with the gas inlet (158) and inner lumens of the gas exchanger fibers on one side of the gas exchanger fibers and a second chamber (164) in fluid communication with the gas outlet (160) and the inner lumens of the gas exchanger fibers on the opposite side of the gas exchanger fibers.

5. The device of claim 1, comprising a first spacer (144) and a second spacer (152) embedded in the potting material body (112), wherein the first spacer (144) is situated between the heat exchanger module (104) and the gaseous micro-emboli removal module (106) and the second spacer (152) is situated between the gaseous micro-emboli removal module (106) and the gas exchanger module (108).

6. The device of claim 1, wherein the blood compartment (114) is a cylindrical blood compartment and the blood flows longitudinally from one end of the cylindrical blood compartment to the other end of the cylindrical blood compartment.

7. The device of claim 1, comprising a blood inlet cap (102) having a blood inlet port (118) and a blood outlet cap (110) having a blood outlet port (122), such that the blood flows in the blood inlet port (118) and through the blood compartment to the blood outlet port (122).

8. The device of claim 1, comprising an arterial blood filter (168) embedded in the potting material body (112) and situated adjacent the gas exchanger module (108).

9. A method of manufacturing a device (100) for conditioning blood, the method comprising:
stacking gas exchanger hollow fiber mat layers (154) in a potting mold;
stacking micro-porous hollow fiber mat layers (146) over the gas exchanger hollow fiber mat layers (154) in the potting mold;
stacking heat exchanger hollow fiber mat layers (124) over the micro-porous hollow fiber mat layers (146) in the potting mold;
wherein at least one of:
the heat exchanger hollow fiber mat layers (124) are alternatively orthogonally angled from one another;
the micro-porous hollow fiber mat layers (146) are alternatively orthogonally angled from one another; and
the gas exchanger hollow fiber mat layers (154) are alternatively orthogonally angled from one another;
spinning the potting mold around a longitudinal axis of the potting mold; and
introducing liquid potting material into the potting mold as the potting mold spins to embed the gas exchanger hollow fiber mat layers (154), the micro-porous hollow fiber mat layers (146) and the heat exchanger hollow fiber mat layers (124) in the potting material and create a blood compartment (114) that extends through the gas exchanger hollow fiber mat layers (154), the micro-porous hollow fiber mat layers (146) and the heat exchanger hollow fiber mat layers (124).

10. The method of claim 9, wherein introducing liquid potting material into the potting mold as the potting mold spins creates a cylindrical blood compartment defined by an inner diameter of the potting material.

11. The method of claim 10, comprising:
removing the potting mold; and
cutting the potting material away to expose ends of heat exchanger hollow fibers in the heat exchanger hollow fiber mat layers (124), ends of micro-porous hollow fibers in the micro-porous hollow fiber mat layers (146) and ends of gas exchanger hollow fibers in the gas exchanger hollow fiber mat layers (154).

12. The method of claim 11, comprising sealing a heat exchanger housing (126) to the potting material and sealing a gaseous micro-emboli removal module housing (148) to the potting material and sealing a gas exchanger housing (156) to the potting material.

13. The method of claim 9, comprising placing a blood outlet cap support (166) in the potting mold followed by placing an arterial blood filter screen layer (168) in the potting mold and then stacking the gas exchanger hollow fiber mat layers (154) into the potting mold prior to the spinning and the introducing steps.

14. The method of claim 9, comprising situating one or more of a first spacer (152) between the gas exchanger hollow fiber mat layers (154) and the micro-porous hollow fiber mat layers (146), a second spacer (144) between the micro-porous hollow fiber mat layers (146) and the heat exchanger hollow fiber mat layers (124), and a blood inlet cap support (140) on the heat exchanger hollow fiber mat layers (124), prior to spinning the potting mold around the longitudinal axis of the potting mold and introducing the liquid potting material into the potting mold.

15. The method of claim 9, comprising one or more of:
knitting a discontinuous weft of a single layer of gas exchanger hollow fibers and cutting the discontinuous weft where the single layer of gas exchanger hollow fibers is missing to provide a gas exchanger hollow fiber mat layer that is stacked into the potting mold;
knitting a discontinuous weft of a single layer of micro-porous hollow fibers and cutting the discontinuous weft where the single layer of micro-porous hollow fibers is missing to provide a micro-porous hollow fiber mat layer that is stacked into the potting mold; and
knitting a discontinuous weft of a single layer of heat exchanger hollow fibers and cutting the discontinuous weft where the single layer of heat exchanger hollow fibers is missing to provide a heat exchanger hollow fiber mat layer that is stacked into the potting mold.

## Patentansprüche

1. Vorrichtung (100) zum Konditionieren von Blut, umfassend:
ein Wärmetauschermodul (104), das eine Wärmetauscher-Faserschicht (124) einschließt, die Wärmetauscherfasern einschließt, die ausgestaltet sind, um ein Wärmetauscherfluid aufzunehmen und Wärme mit dem Blut auszutauschen;
ein Entfernungsmodul (106) für gasförmige Mikroemboli, das eine mikroporöse Faserschicht (146) einschließt, die mikroporöse Fasern einschließt, die ausgestaltet sind, um atmosphärische oder subatmosphärische Drücke aufzunehmen, so dass mindestens einige gasförmige Mikroemboli durch die mikroporösen Fasern aus dem Blut gezogen werden;
ein Gastauschermodul (108), das eine Gastauscher-Faserschicht (154) einschließt, die Gastauscherfasern einschließt, die ausgestaltet sind, um eine Gasmischung aufzunehmen und Gas mit dem Blut auszutauschen; und
einen Vergussmaterialkörper (112), der die Wärmetauscherfasern, die mikroporösen Fasern und die Gastauscherfasern einbettet und ein Blutkompartiment (114) definiert, das sich durch das Wärmetauschermodul (104), das Entfernungsmodul (106) für gasförmige Mikroemboli und das Gastauschermodul (108) hindurch erstreckt;
**gekennzeichnet durch** mindestens eines der folgenden:
das Wärmetauschermodul (104) schließt gestapelte Wärmetauscher-Hohlfasermattenschichten (124) ein, wobei die Wärmetauscher-Hohlfasermattenschichten (124) alternierend orthogonal zueinander gewinkelt sind;
das Entfernungsmodul (106) für gasförmige Mikroemboli schließt gestapelte mikroporöse Hohlfasermattenschichten (146) ein, wobei die mikroporösen Hohlfasermattenschichten (146) alternierend orthogonal zueinander gewinkelt sind; und
das Gastauschermodul (108) schließt gestapelte Gastauscher-Hohlfasermattenschichten (154) ein, wobei die Gastauscher-Hohlfasermattenschichten (154) alternierend orthogonal zueinander gewinkelt sind.

2. Vorrichtung nach Anspruch 1, umfassend ein Wärmetauschergehäuse (126), das an den Vergussmaterialkörper (112) gesiegelt ist und einen Einlass (128) und einen Auslass (130) aufweist, wobei das Wärmetauschergehäuse (126) eine erste Kammer (132) in Fließkommunikation mit dem Einlass (128) und Innenlumina der Wärmetauscherfasern auf einer Seite der Wärmetauscherfasern und eine zweite Kammer (134) in Fließkommunikation mit dem Auslass (130) und den Innenlumina der Wärmetauscherfasern auf der entgegengesetzten Seite der Wärmetauscherfasern bereitstellt.

3. Vorrichtung nach Anspruch 1, umfassend ein Entfernungsmodulgehäuse (148) für gasförmige Mikroemboli, das an den Vergussmaterialkörper (112) gesiegelt ist und einen Fluidauslass (150) in Fließkommunikation mit mindestens Innenlumina der mikroporösen Fasern auf einer Seite der mikroporösen Fasern aufweist.

4. Vorrichtung nach Anspruch 1, umfassend ein Gastauschergehäuse (156), das an den Vergussmaterialkörper (112) gesiegelt ist und einen Gaseinlass (158) und einen Gasauslass (160) aufweist, wobei das Gastauschergehäuse (156) eine erste Kammer (162) in Fließkommunikation mit dem Gaseinlass (158) und Innenlumina der Gastauscherfasern auf einer Seite der Gastauscherfasern und eine zweite Kammer (164) in Fließkommunikation mit dem Gasauslass (160) und den Innenlumina der Gastauscherfasern auf der entgegengesetzten Seite der Gastauscherfasern bereitstellt.

5. Vorrichtung nach Anspruch 1, umfassend einen ersten Abstandhalter (144) und einen zweiten Abstandhalter (152) eingebettet in den Vergussmaterialkörper (112), wobei sich der erste Abstandhalter (144) zwischen dem Wärmetauschermodul (104) und dem Entfernungsmodul (106) für gasförmige Mikroemboli befindet und sich der zweite Abstandhalter (152) zwischen dem Entfernungsmodul (106) für gasförmige Mikroemboli und dem Gastauschermodul (108) befindet.

6. Vorrichtung nach Anspruch 1, wobei das Blutkompartiment (114) ein zylindrisches Blutkompartiment ist und das Blut in Längsrichtung von einem Ende des zylindrischen Blutkompartiments zu dem anderen Ende des zylindrischen Blutkompartiments fließt.

7. Vorrichtung nach Anspruch 1, umfassend eine Bluteinlasskappe (102), die einen Bluteinlassport (118) aufweist, und eine Blutauslasskappe (110), die einen Blutauslassport (122) aufweist, so dass das Blut in den Bluteinlassport (118) und durch das Blutkompartiment hindurch zu dem Blutauslassport (122) fließt.

8. Vorrichtung nach Anspruch 1, umfassend einen arteriellen Blutfilter (168), der in den Vergussmaterialkörper (112) eingebettet ist und sich benachbart zu dem Gastauschermodul (108) befindet.

9. Verfahren zur Fertigung einer Vorrichtung (100) zum Konditionieren von Blut, wobei das Verfahren umfasst:
Stapeln von Gastauscher-Hohlfasermattenschichten (154) in einem Vergussformwerkzeug;
Stapeln von mikroporösen Hohlfasermattenschichten (146) über den Gastauscher-Hohlfasermattenschichten (154) in dem Vergussformwerkzeug;
Stapeln von Wärmetauscher-Hohlfasermattenschichten (124) über den mikroporösen Hohlfasermattenschichten (146) in dem Vergussformwerkzeug;
wobei mindestens eines der folgenden zutrifft:
die Wärmetauscher-Hohlfasermattenschichten (124) sind alternierend orthogonal zueinander gewinkelt;
die mikroporösen Hohlfasermattenschichten (146) sind alternierend orthogonal zueinander gewinkelt;
die Gastauscher-Hohlfasermattenschichten (154) sind alternierend orthogonal zueinander gewinkelt;
Drehen des Vergussformwerkzeugs um eine Längsachse des Vergussformwerkzeugs; und
Einbringen von flüssigem Vergussmaterial in das Vergussformwerkzeug, wenn sich das Vergussformwerkzeug dreht, um die Gastauscher-Hohlfasermattenschichten (154), die mikroporösen Hohlfasermattenschichten (146) und die Wärmetauscher-Hohlfasermattenschichten (124) in dem Vergussmaterial einzubetten und ein Blutkompartiment (114) zu erzeugen, das sich durch die Gastauscher-Hohlfasermattenschichten (154), die mikroporösen Hohlfasermattenschichten (146) und die Wärmetauscher-Hohlfasermattenschichten (124) hindurch erstreckt.

10. Verfahren nach Anspruch 9, wobei Einbringen von flüssigem Vergussmaterial in das Vergussformwerkzeug, wenn sich das Vergussformwerkzeug dreht, ein zylindrisches Blutkompartiment erzeugt, das durch einen Innendurchmesser des Vergussmaterials definiert ist.

11. Verfahren nach Anspruch 10, umfassend:
Entfernen des Vergussformwerkzeugs; und
Wegschneiden des Vergussmaterials, um Enden der Wärmetauscherhohlfasern in den Wärmetauscher-Hohlfasermattenschichten (124), Enden von mikroporösen Hohlfasern in den mikroporösen Hohlfasermattenschichten (146) und Enden der Gastauscherhohlfasern in den Gastauscher-Hohlfasermattenschichten (154) zu exponieren.

12. Verfahren nach Anspruch 11, umfassend Siegeln eines Wärmetauschergehäuses (126) an das Vergussmaterial und Siegeln eines Entfernungsmodulgehäuses (148) für gasförmige Mikroemboli an das Vergussmaterial und Siegeln eines Gastauschergehäuses (156) an das Vergussmaterial.

13. Verfahren nach Anspruch 9, umfassend Platzieren eines Blutauslasskappenträgers (166) in dem Vergussformwerkzeug, gefolgt von Platzieren einer Siebschicht (168) des arteriellen Blutfilters in dem Vergussformwerkzeug und dann Stapeln der Gastauscher-Hohlfasermattenschichten (154) in dem Vergussformwerkzeug vor den Dreh- und den Einbringungsschritten.

14. Verfahren nach Anspruch 9, umfassend Aufstellen von einem oder mehreren von einem ersten Abstandhalter (152) zwischen den Gastauscher-Hohlfasermattenschichten (154) und den mikroporösen Hohlfasermattenschichten (146), einem zweiten Abstandhalter (144) zwischen den mikroporösen Hohlfasermattenschichten (146) und den Wärmetauscher-Hohlfasermattenschichten (124), und einem Bluteinlasskappenträger (140) auf den Wärmetauscher-Hohlfasermattenschichten (124) vor dem Drehen des Vergussformwerkzeugs um die Längsachse des Vergussformwerkzeugs und Einbringen des flüssigen Vergussmaterials in das Vergussformwerkzeug.

15. Verfahren nach Anspruch 9, umfassend ein oder mehrere der folgenden:
Stricken eines diskontinuierlichen Schussfadens einer Einzelschicht aus Gastauscherhohlfasern und Schneiden des diskontinuierlichen Schussfadens, wo die Einzelschicht aus Gastauscherhohlfasern fehlt, um eine Gastauscher-Hohlfasermattenschicht bereitzustellen, die in das Vergussformwerkzeug gestapelt wird;
Stricken eines diskontinuierlichen Schussfadens einer Einzelschicht aus mikroporösen Hohlfasern und Schneiden des diskontinuierlichen Schussfadens, wo die Einzelschicht aus mikroporösen Hohlfasern fehlt, um eine mikroporöse Hohlfasermattenschicht bereitzustellen, die in das Vergussformwerkzeug gestapelt wird; und
Stricken eines diskontinuierlichen Schussfadens einer Einzelschicht aus Wärmetauscherhohlfasern und Schneiden des diskontinuierlichen Schussfadens, wo die Einzelschicht aus Wärmetauscherhohlfasern fehlt, um eine Wärmetauscher-Hohlfasermattenschicht bereitzustellen, die in das Vergussformwerkzeug gestapelt wird.

## Revendications

1. Dispositif (100) de conditionnement du sang comprenant :
un module d'échangeur de chaleur (104) comprenant une couche de fibres d'échangeur de chaleur (124) comprenant des fibres d'échangeur de chaleur configurées pour recevoir un fluide d'échangeur de chaleur et échanger de la chaleur avec le sang ;
un module d'élimination de micro-emboles gazeux (106) comprenant une couche de fibres microporeuses (146) comprenant des fibres microporeuses configurées pour recevoir des pressions atmosphériques ou subatmosphériques de sorte qu'au moins certains micro-emboles gazeux soient extraits du sang à travers les fibres microporeuses ;
un module d'échangeur de gaz (108) comprenant une couche de fibres d'échangeur de gaz (154) comprenant des fibres d'échangeur de gaz configurées pour recevoir un mélange gazeux et échanger du gaz avec le sang ; et
un corps de matériau d'enrobage (112) qui intègre les fibres d'échangeur de chaleur, les fibres microporeuses et les fibres d'échangeur de gaz et définit un compartiment sanguin (114) qui s'étend à travers le module d'échangeur de chaleur (104), le module d'élimination de micro-emboles gazeux (106) et le module d'échangeur de gaz (108) ;
**caractérisé par** au moins l'un parmi :
le module d'échangeur de chaleur (104) comprenant des couches de mats de fibres creuses d'échangeur de chaleur empilées (124), où les couches de mats de fibres creuses d'échangeur de chaleur (124) sont alternativement inclinées orthogonalement les unes par rapport aux autres ;
le module d'élimination de micro-emboles gazeux (106) comprenant des couches de mats de fibres creuses microporeuses empilées (146), où les couches de mats de fibres creuses microporeuses (146) sont alternativement inclinées orthogonalement les unes par rapport aux autres ; et
le module d'échangeur de gaz (108) comprenant des couches de mats de fibres creuses d'échangeur de gaz empilées (154), où les couches de mats de fibres creuses d'échangeur de gaz (154) sont alternativement inclinées orthogonalement les unes par rapport aux autres.

2. Dispositif selon la revendication 1, comprenant un boîtier d'échangeur de chaleur (126) scellé au corps de matériau d'enrobage (112) et ayant une entrée (128) et une sortie (130), où le boîtier d'échangeur de chaleur (126) fournit une première chambre (132) en communication fluidique avec l'entrée (128) et des lumières internes des fibres d'échangeur de chaleur sur un côté des fibres d'échangeur de chaleur et une seconde chambre (134) en communication fluidique avec la sortie (130) et les lumières internes des fibres d'échangeur de chaleur sur le côté opposé des fibres d'échangeur de chaleur.

3. Dispositif selon la revendication 1, comprenant un boîtier de module d'élimination de micro-emboles gazeux (148) scellé au corps de matériau d'enrobage (112) et ayant une sortie de fluide (150) en communication fluidique avec au moins des lumières internes des fibres microporeuses sur un côté des fibres microporeuses.

4. Dispositif selon la revendication 1, comprenant un boîtier d'échangeur de gaz (156) scellé au corps de matériau d'enrobage (112) et ayant une entrée de gaz (158) et une sortie de gaz (160), où le boîtier d'échangeur de gaz (156) fournit une première chambre (162) en communication fluidique avec l'entrée de gaz (158) et des lumières internes des fibres d'échangeur de gaz sur un côté des fibres d'échangeur de gaz et une seconde chambre (164) en communication fluidique avec la sortie de gaz (160) et les lumières internes des fibres d'échangeur de gaz sur le côté opposé des fibres d'échangeur de gaz.

5. Dispositif selon la revendication 1, comprenant un premier élément d'espacement (144) et un second élément d'espacement (152) intégrés dans le corps de matériau d'enrobage (112), où le premier élément d'espacement (144) est situé entre le module d'échangeur de chaleur (104) et le module d'élimination de micro-emboles gazeux (106) et le second élément d'espacement (152) est situé entre le module d'élimination de micro-emboles gazeux (106) et le module d'échangeur de gaz (108).

6. Dispositif selon la revendication 1, dans lequel le compartiment sanguin (114) est un compartiment sanguin cylindrique et le sang s'écoule longitudinalement d'une extrémité du compartiment sanguin cylindrique à l'autre extrémité du compartiment sanguin cylindrique.

7. Dispositif selon la revendication 1, comprenant un capuchon d'entrée de sang (102) ayant un orifice d'entrée de sang (118) et un capuchon de sortie de sang (110) ayant un orifice de sortie de sang (122), de sorte que le sang s'écoule dans l'orifice d'entrée de sang (118) et à travers le compartiment sanguin vers l'orifice de sortie de sang (122).

8. Dispositif selon la revendication 1, comprenant un filtre de sang artériel (168) intégré dans le corps de matériau d'enrobage (112) et situé de manière adjacente au module d'échangeur de gaz (108).

9. Procédé de fabrication d'un dispositif (100) de conditionnement du sang, le procédé comprenant :
l'empilement de couches de mats de fibres creuses d'échangeur de gaz (154) dans un moule d'enrobage ;
l'empilement de couches de mats de fibres creuses microporeuses (146) sur les couches de mats de fibres creuses d'échangeur de gaz (154) dans le moule d'enrobage ;
l'empilement de couches de mats de fibres creuses d'échangeur de chaleur (124) sur les couches de mats de fibres creuses microporeuses (146) dans le moule d'enrobage ;
dans lequel au moins l'un parmi :
les couches de mats de fibres creuses d'échangeur de chaleur (124) sont alternativement inclinées orthogonalement les unes par rapport aux autres ;
les couches de mats de fibres creuses microporeuses (146) sont alternativement inclinées orthogonalement les unes par rapport aux autres ; et
les couches de mats de fibres creuses d'échangeur de gaz (154) sont alternativement inclinées orthogonalement les unes par rapport aux autres ;
la rotation du moule d'enrobage autour d'un axe longitudinal du moule d'enrobage ; et
l'introduction d'un matériau d'enrobage liquide dans le moule d'enrobage à mesure que le moule d'enrobage tourne pour intégrer les couches de mats de fibres creuses d'échangeur de gaz (154), les couches de mats de fibres creuses microporeuses (146) et les couches de mats de fibres creuses d'échangeur de chaleur (124) dans le matériau d'enrobage et créer un compartiment sanguin (114) qui s'étend à travers les couches de mats de fibres creuses d'échangeur de gaz (154), les couches de mats de fibres creuses microporeuses (146) et les couches de mats de fibres creuses d'échangeur de chaleur (124).

10. Procédé selon la revendication 9, dans lequel l'introduction d'un matériau d'enrobage liquide dans le moule d'enrobage à mesure que le moule d'enrobage tourne crée un compartiment sanguin cylindrique défini par un diamètre interne du matériau d'enrobage.

11. Procédé selon la revendication 10, comprenant :
le retrait du moule d'enrobage ; et
la découpe du matériau d'enrobage pour exposer les extrémités des fibres creuses d'échangeur de chaleur dans les couches de mats de fibres creuses d'échangeur de chaleur (124), les extrémités des fibres creuses microporeuses dans les couches de mats de fibres creuses microporeuses (146) et les extrémités des fibres creuses d'échangeur de gaz dans les couches de mats de fibres creuses d'échangeur de gaz (154).

12. Procédé selon la revendication 11, comprenant le scellement d'un boîtier d'échangeur de chaleur (126) au matériau d'enrobage et le scellement d'un boîtier de module d'élimination de micro-emboles gazeux (148) au matériau d'enrobage et le scellement d'un boîtier d'échangeur de gaz (156) au matériau d'enrobage.

13. Procédé selon la revendication 9, comprenant le placement d'un support de capuchon de sortie de sang (166) dans le moule d'enrobage suivi du placement d'une couche de tamis de filtre de sang artériel (168) dans le moule d'enrobage et ensuite l'empilement des couches de mats de fibres creuses d'échangeur de gaz (154) dans le moule d'enrobage avant les étapes de rotation et d'introduction.

14. Procédé selon la revendication 9, comprenant la mise en place d'un ou de plusieurs parmi un premier élément d'espacement (152) entre les couches de mats de fibres creuses d'échangeur de gaz (154) et les couches de mats de fibres creuses microporeuses (146), un second élément d'espacement (144) entre les couches de mats de fibres creuses microporeuses (146) et les couches de mats de fibres creuses d'échangeur de chaleur (124), et un support de capuchon d'entrée de sang (140) sur les couches de mats de fibres creuses d'échangeur de chaleur (124), avant la rotation du moule d'enrobage autour de l'axe longitudinal du moule d'enrobage et l'introduction du matériau d'enrobage liquide dans le moule d'enrobage.

15. Procédé selon la revendication 9, comprenant un ou plusieurs parmi :
le tricotage d'une trame discontinue d'une seule couche de fibres creuses d'échangeur de gaz et la coupe de la trame discontinue à l'endroit où la seule couche de fibres creuses d'échangeur de gaz est manquante pour fournir une couche de mats de fibres creuses d'échangeur de gaz qui est empilée dans le moule d'enrobage ;
le tricotage d'une trame discontinue d'une seule couche de fibres creuses microporeuses et la coupe de la trame discontinue à l'endroit où la seule couche de fibres creuses microporeuses est manquante pour fournir une couche de mats de fibres creuses microporeuses qui est empilée dans le moule d'enrobage ; et
le tricotage d'une trame discontinue d'une seule couche de fibres creuses d'échangeur de chaleur et la coupe de la trame discontinue à l'endroit où la seule couche de fibres creuses d'échangeur de chaleur est manquante pour fournir une couche de mats de fibres creuses d'échangeur de chaleur qui est empilée dans le moule d'enrobage.
